# EUROPÄISCHE PATENTSCHRIFT

(11) **EP 1 384 451 B1**
(45) Veröffentlichungstag und Bekanntmachung des Hinweises auf die Patenterteilung: **05.01.2011**
(21) Anmeldenummer: 03015922.2
(22) Anmeldetag: 12.07.2003
(51) Int. Cl.: A61F 2/18

(54) **Steigbügelprothese**
Stapedial prosthesis
Prothèse d'étrier

(30) Priorität: 23.07.2002 DE 20211102 U; 21.08.2002 DE 20212771 U
(43) Veröffentlichungstag der Anmeldung: 28.01.2004
(73) Patentinhaber: Heinz Kurz GmbH Medizintechnik, 72144 Dusslingen (DE)
(72) Erfinder: Awengen, Daniel F., Dr. med., 4102 Binningen (CH); Steinhardt, Uwe, 72108 Rottenburg (DE); Kurz, Heinz, 72144 Dusslingen (DE)
(74) Vertreter: Möbus, Daniela

(56) Entgegenhaltungen:
- EP-A- 1 073 313
- DE-C- 19 931 788
- DE-U- 29 609 687

## Beschreibung

Die Erfindung betrifft eine Steigbügelprothese zur Implantation im Mittelohr mit einem auf den langen Ambossfortsatz aufschiebbaren elastischen Klipp in Form einer einseitig offenen Klammer.

Eine solche Steigbügelprothese ist im deutschen Gebrauchsmuster DE 296 09 687.3 der Anmelderin bereits beschrieben. Diese bekannte Prothese zeichnet sich dadurch aus, dass sie sich durch einfaches Aufklippen auf den langen Ambossfortsatz relativ leicht implantieren lässt. Sie hält allein durch die Klemmwirkung des Klipp. Weitere Befestigungsmittel sind überflüssig. Dennoch kann es im Verlauf der Implantation zu Verkippungen der Prothese kommen, wodurch die Operation für den Chirurgen erschwert wird. EP-A-1073313 beschreibt eine Steigbügelprothese mit den technischen Merkmalen aus dem Oberbegriff aus Anspruch 1.

Der vorliegenden Erfindung liegt daher die Aufgabe zugrunde, die bekannte Steigbügelprothese dahin gehend zu verbessern, dass ihre Implantation noch einfacher wird.

Die Aufgabe wird mit einer Steigbügelprothese der eingangs genannten Art erfindungsgemäß durch die technischen Merkmale aus Anspruch 1 gelöst. Durch das Aufhängen der Prothese am langen Ambossfortsatz gewinnt der Operateur die Möglichkeit, das Operationsbesteck zu wechseln und z. B. ein Häkchen zum Aufschieben der Prothese auf den langen Ambossfortsatz zu verwenden.

Die Handhabung der Prothese lässt sich außerdem noch dadurch erleichtern, dass sie auf ihrer Außenseite mit einem Nippel zum Aufschieben der Prothese auf den langen Ambossfortsatz versehen sein kann. An diesem Nippel kann der Operateur mit einer Pinzette oder einem Haken angreifen.

Anstelle eines Nippels kann die Klammer auch auf ihrer der Öffnung gegenüberliegenden Seite mit einer Kerbe zum Aufschieben der Prothese auf den langen Ambossfortsatz versehen sein. Auch an dieser Kerbe kann ein entsprechendes Instrument angesetzt werden.

Weitere Vorteile ergeben sich durch den Klipp, der den langen Ambossfortsatz nicht vollständig umgreift. Dadurch kann die Ausbildung von Einschnürungen am langen Ambossfortsatz und damit das Auftreten von potentiellen Nekrosen verhindert werden. Besonders vorteilhaft ist es dabei, wenn nach der Implatation der Klipp so am langen Ambossfortsatz angeordnet ist, dass er in zwei Bereichen des Umfangs des langen Ambossfortsatzes nicht an diesem anliegt. Damit werden die Versorgungsgefäße am langen Ambossfortsatz nur in den Anlagebereichen tangiert. Die übrigen Gefäße verlaufen in den beiden Bereichen, in denen die Klammer nicht am Ambossfortsatz anliegt, sodass die Nährstoffversorgung des langen Ambossfortsatzes und des Proc. Lenticularis nicht gefährdet wird.

Die Prothese kann aus jedem biologisch verträglichen Material gefertigt sein. Besondere Vorteile ergeben sich, wenn mindestens der Klipp aus Titan gefertigt ist, da dieses Material eine hervorragende Biokompatibilität im menschlichen Mittelohr aufweist.

Nachfolgend werden bevorzugte Ausführungsbeispiele erfindungsgemäßer Prothesen anhand der Zeichnung näher beschrieben.

Es zeigen:
- Fig. 1: eine Seitenansicht einer ersten Stapes- prothese;
- Fig. 2: eine Seitenansicht einer zweiten Stapes- prothese;
- Fig. 3: eine Seitenansicht einer dritten Stapes- prothese.

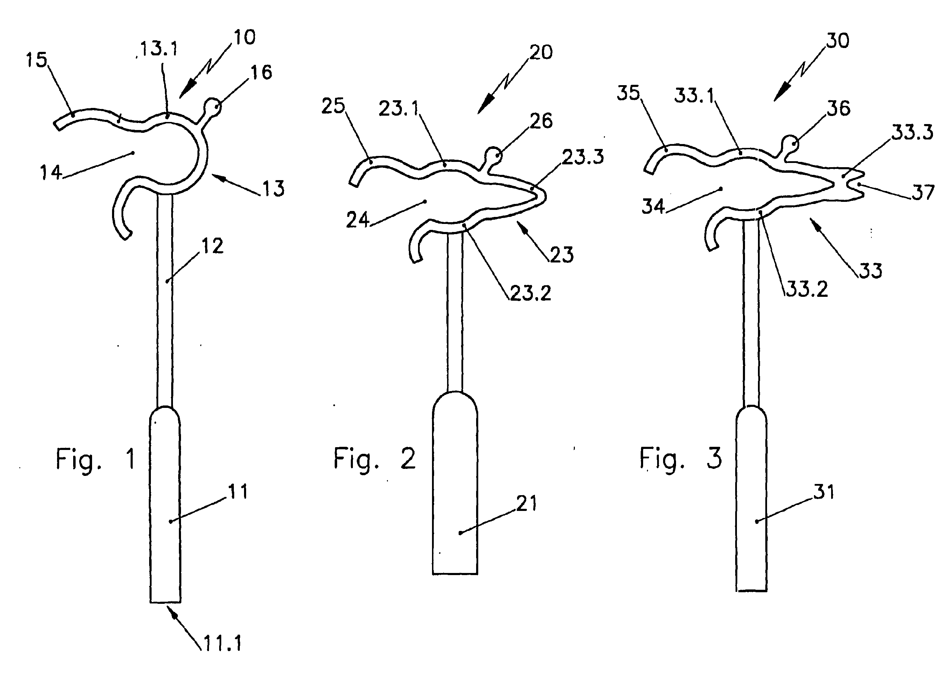

Die in Fig. 1 gezeigte Steigbügelprothese 10 wird von einem kolbenförmigen Teil 11 gebildet, der mit seiner unteren Stirnseite 11.1 im ovalen Fenster sitzt und somit den Schall zum Innenohr überträgt. Am oberen Ende des kolbenförmigen Teils 11 schließt sich ein Prothesenschaft 12 an, der an seinem oberen Ende einen Klipp 13 aus einem elastischen Material aufweist. Der Klipp 13 ist dabei in Form einer einseitig offenen Klammer ausgebildet. Er kann mit seiner Öffnung 14 über den langen Ambossfortsatz aufgeschoben werden. Der obere Schenkel 13.1 der Klammer 13 ist über die Öffnung 14 hinaus verlängert und bildet dort einen Bogen 15, mit dem die gesamte Prothese 10 am langen Ambossfortsatz aufgehängt werden kann. Zur Erleichterung des Aufschiebens ist an der Außenseite der Klammer 13 ein Nippel 16 angeordnet, an dem der Operateur beispielsweise mit einer Pinzette oder einem Haken angreifen kann.

Während der Klipp 13 der Prothese 10 derart ausgebildet ist, dass er den Ambossfortsatz dreiviertelkreisförmig umschließt, weist die Prothese 20 aus Fig. 2 einen Klipp 23 auf, der den langen Ambossfortsatz nur in zwei Bereichen 23.1 und 23.2 berührt. Der dazwischenliegende Bereich 23.3 verläuft in einem relativ großen Abstand zum Ambossfortsatz, sodass dort die Versorgungsgefäße des langen Ambossfortsatzes nicht durch den Klipp 23 abgeschnürt werden können. Ansonsten ist der Klipp 23 ähnlich wie der Klipp 13 ausgebildet. Der obere Schenkel weist wieder einen bogenförmigen Abschnitt 25 auf, mit dem die Prothese 20 am langen Ambossfortsatz aufgehängt werden kann. Außerdem ist auf der Außenseite des Klipps 23 wieder ein Nippel für das Ansetzen eines Operationsbestecks vorgesehen.

Die Prothese 30 aus Fig. 3 weist einen Klipp 33 auf, der sehr ähnlich wie der Klipp 23 aus Fig. 2 geformt ist. Zusätzlich zu dem Nippel 36 ist jetzt jedoch in dem der Öffnung 34 gegenüberliegenden Bereich 33.3 des Klipps 33 eine Kerbe 37 vorgesehen, in der ebenfalls ein Operationsbesteck angesetzt werden kann, um die Prothese 30 auf den langen Ambossfortsatz aufzuschieben.

Die Prothesen 10 bis 30 unterscheiden sich außerdem in ihrer Länge und auch in der Dicke der kolbenförmigen Teile 11, 21 und 31.

## Patentansprüche

1. Steigbügelprothese(10; 20; 30) zur Implantation im Mittelohr mit einem Prothesenschaft (12; 22; 32) und einem daran befestigten, auf den langen Ambossfortsatz aufschiebbaren elastischen Klipp in Form einer einseitig offenen Klammer (13; 23; 33), die zwei gegenüber liegende, im implantierten Zustand der Prothese (10; 20; 30) um den langen Ambossfortsatz herum gekrümmte Schenkel (13.1, 23.1, 23.2; 33.1, 33.2) aufweist, welche eine Öffnung (14; 24; 34) zwischen sich frei lassen, wobei die Klammer (13; 23; 33) mit mindestens einem ersten Schenkel (13.1; 23.1; 33.1) durch einen den ersten Schenkel (13.1; 23.1; 33.1) fortsetzenden und sich in Schenkelrichtung über die Öffnung (14; 24; 34) nach außen erstreckenden Bogen (15; 25; 35) verlängert ist,
**dadurch gekennzeichnet,**
**dass** die Klammer (13; 23; 33) an dem ihrem ersten Schenkel (13.1; 23.1; 33.1) gegenüber liegenden zweiten Schenkel (23.2; 33.2) mit dem Prothesenschaft (12; 22; 32) derart verbunden ist, dass der Prothesenschaft (12; 22; 32) in der Klammerebene liegt und ausgehend vom zweiten Schenkel (23.2; 33.2) radial von der Klammer (13; 23; 33) weg nach außen verläuft,
und **dass** der Bogen (15; 25; 35) in die gleiche Richtung gekrümmt ist, wie der erste Schenkel (13.1; 23.1; 33.1), so dass die Prothese (10; 20; 30) mit dem Bogen (15; 25; 35) am langen Ambossfortsatz des menschlichen Mittelohrs vor dem Aufschieben aufhängbar ist.

2. Prothese nach Anspruch 1, **dadurch gekennzeichnet, dass** die Klammer (13, 23, 33) auf ihrer Außenseite mit einem Nippel (16, 26, 36) zum Aufschieben der Prothese auf den langen Ambossfortsatz versehen ist.

3. Prothese nach Anspruch 1 oder 2, **dadurch gekennzeichnet, dass** die Klammer (13, 23, 33) auf ihrer der Öffnung (14, 24, 34) gegenüberliegenden Seite (33.3) mit einer Kerbe (37) zum Aufschieben der Prothese auf den langen Ambossfortsatz versehen ist.

4. Prothese nach einem der Ansprüche 1 bis 3, **dadurch gekennzeichnet, dass** nach der Implantation der Klipp (23, 33) so am langen Ambossfortsatz angeordnet ist, dass er nur in zwei Bereichen (23.1, 23.2, 33.1, 33.2) des Umfangs des langen Ambossfortsatzes an diesem anliegt.

5. Prothese nach einem der Ansprüche 1 bis 4, **dadurch gekennzeichnet, dass** mindestens der Klipp (13, 23, 33) aus Titan gefertigt ist.

## Claims

1. A stapes prosthesis (10; 20; 30), for implantation in the middle ear, comprising a prosthesis shaft (12; 22; 32) with an attached resilient clip which can be pushed onto the long process of the incus, said resilient clip taking the form of a clamp (13; 23; 33) which is open at one side and has two opposing legs (13.1; 23.1, 23.2; 33.1, 33.2) which are curved around the long process of the incus in the implanted state of the prosthesis (10; 20; 30), and have an opening (14; 24; 34) between them, in which case the clamp (13; 23; 33) having at least a first leg (13.1; 23.1; 33.1) is extended by an arch (15; 25; 35) in continuation of said first leg (13.1; 23.1; 33.1) and extending outwards in direction of the leg over the opening (14; 24; 34),
**characterized in that**
the clamp (13; 23; 33) is attached at the second leg (23.2; 33.2) located opposite its first leg (13.1; 23.1; 33.1) to the prosthesis shaft (12; 22; 32) in such a way that the prosthesis shaft (12; 22; 32) lies in the plane of the clamp and, starting from the second leg (23.2; 33.2), extends radially outwards away from the clamp (13; 23; 33),
and **in that** the arch (15; 25; 35) is curved in the same direction as the first leg (13.1; 23.1; 33.1) so that the prosthesis (10; 20; 30) can be suspended via the arch (15; 25; 35) on the long process of the incus of the human middle ear prior to being pushed into place.

2. A prosthesis according to Claim 1, **characterized in that** the clamp (13, 23, 33) is provided on the outside with a nipple (16, 26, 36) to aid pushing of the prosthesis onto the long process of the incus.

3. A prosthesis according to Claim 1 or 2, **characterized in that** the clamp (13, 23, 33) is provided on its side (33.3) lying opposite the opening (14, 24, 34) with a notch (37) to aid pushing of the prosthesis onto the long process of the incus.

4. A prosthesis according to one of Claims 1 to 3, **characterized in that**, after implantation, the clip (23, 33) is placed on the long process of the incus in such a way that, only in two areas (23.1, 23.2, 33.1, 33.2) of the perimeter of the long process of the incus, it does rest against the latter.

5. A prosthesis according to one of Claims 1 to 4, **characterized in that** at least the clip (13, 23, 33) is made of titanium.

## Revendications

1. Prothèse d'étrier (10 ; 20 ; 30) pour implantation dans l'oreille moyenne, comprenant une tige de prothèse (12 ; 22 ; 32) et un clip élastique qui y est fixé et peut coulisser sur le long prolongement de l'enclume sous la forme d'une agrafe (13 ; 23 ; 33) ouverte d'un côté, qui présente deux branches (13.1, 23.1, 23.2 ; 33.1, 33.2) en regard l'une de l'autre et courbées à l'état implanté de la prothèse (10 ; 20 ; 30) autour du long prolongement de l'enclume, lesquelles branches laissent libre entre elles une ouverture (14 ; 24 ; 34), dans laquelle l'agrafe (13 ; 23 ; 33) est prolongée, sur au moins une première branche (13.1 ; 23.1 ; 33.1), par un arc (14 ; 24 ; 34) prolongeant la première branche (13.1 ; 23.1 ; 33.1) et s'étendant vers l'extérieur dans la direction de la branche au-dessus de l'ouverture (14 ; 24 ; 34),
**caractérisée en ce que**
l'agrafe (13 ; 23 ; 33) est raccordée, sur sa seconde branche (23.2 ; 33.2) opposée à sa première branche (13.1 ; 23.1 ; 33.1), à la tige de prothèse (12 ; 22 ; 32) de sorte que la tige de prothèse (12 ; 22 ; 32) se situe dans le plan de l'agrafe et s'étende de la seconde branche (23.2 ; 33.2) radialement vers l'extérieur en s'écartant de l'agrafe (13 ; 23 ; 33),
et **en ce que** l'arc (15 ; 25 ; 35) est courbé dans la même direction que celle de la première branche (13.1 ; 23.1 ; 33.1) de sorte que la prothèse (10 ; 20 ; 30) puisse être suspendue avec l'arc (15 ; 25 ; 35) sur le long prolongement de l'enclume de l'oreille moyenne humaine avant son coulissement.

2. Prothèse selon la revendication 1, **caractérisée en ce que** l'agrafe (13, 23, 33) est pourvue, sur son côté externe, d'unnipple (16, 26, 36) pour faire coulisser la prothèse sur le long prolongement de l'enclume.

3. Prothèse selon la revendication 1 ou 2, **caractérisée en ce que** l'agrafe (13, 23, 33) est dotée, sur son côté (33.3) opposé à l'ouverture (14, 24, 34), d'une encoche (37) pour faire coulisser la prothèse sur le long prolongement de l'enclume.

4. Prothèse selon l'une quelconque des revendications 1 à 3, **caractérisée en ce qu'**après l'implantation, le clip (23, 33) est agencé sur le long prolongement de l'enclume de sorte qu'il s'appuie seulement sur celui-ci dans deux zones (23.1, 23.2, 33.1, 33.2) du pourtour du long prolongement de l'enclume.

5. Prothèse selon l'une quelconque des revendications 1 à 4, **caractérisée en ce qu'**au moins le clip (13, 23, 33) est fabriqué en titane.
